(19) Europäisches Patentamt
European Patent Office
Office européen des brevets

(11) EP 0 629 211 B1

(12) **EUROPÄISCHE PATENTSCHRIFT**

(45) Veröffentlichungstag und Bekanntmachung des Hinweises auf die Patenterteilung:
**12.08.1998 Patentblatt 1998/33**

(21) Anmeldenummer: **93904010.1**

(22) Anmeldetag: **24.02.1993**

(51) Int Cl.[6]: **C07K 5/06**, C07K 5/08, C07K 5/10, A61K 38/04

(86) Internationale Anmeldenummer:
**PCT/EP93/00424**

(87) Internationale Veröffentlichungsnummer:
**WO 93/18057 (16.09.1993 Gazette 1993/22)**

(54) **4-OXO-2-THIOXOIMIDAZOLIDIN-DERIVATE ALS HEMMSTOFFE DER BLUTPLÄTTCHENAGGREGATION**

4-OXO-2-THIOXOIMIDAZOLIDINE DERIVATES AS INHIBITORS OF BLOOD PLATELET AGGREGATION

DERIVES DE 4-OXO-2-THIOXOIMIDAZOLIDINE UTILISES COMME INHIBITEURS DE L'AGREGATION PLAQUETTAIRE SANGUINE

(84) Benannte Vertragsstaaten:
**AT BE CH DE DK ES FR GB IT LI NL SE**

(30) Priorität: **07.03.1992 DE 4207254**

(43) Veröffentlichungstag der Anmeldung:
**21.12.1994 Patentblatt 1994/51**

(73) Patentinhaber: **HOECHST AKTIENGESELLSCHAFT**
**65926 Frankfurt am Main (DE)**

(72) Erfinder:
- **ZOLLER, Gerhard**
  **D-6369 Schöneck (DE)**
- **JABLONKA, Bernd**
  **D-6232 Bad Soden (DE)**
- **KNOLLE, Jochen**
  **D-6239 Kriftel (DE)**
- **JUST, Melitta**
  **D-6070 Langen (DE)**
- **KÖNIG, Wolfgang**
  **D-94375 Stallwang (DE)**

(56) Entgegenhaltungen:
**EP-A- 0 449 079** **US-A- 4 970 225**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft 4-Oxo-2-thioxoimidazolidin-Derivate, ihre Herstellung und ihre Verwendung als Hemmstoffe der Blutplättchenaggregation.

In der EP-A 449 079, sowie in der unveröffentlichten deutschen Patentanmeldung P 41 26 277.8 sind Hydantoinderivate mit thrombozytenaggregationshemmender Wirkung beschrieben. Weitere Forschungsarbeiten haben gezeigt, daß auch die Verbindungen der vorliegenden Erfindung starke Hemmstoffe der Blutplättchenaggregation sind.

Die vorliegende Erfindung betrifft Verbindungen der allgemeinen Formel I

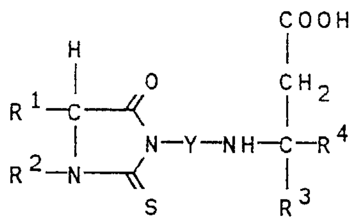

(I)

worin

Y $-(CH_2)_m-CO-$, wobei m für 1 oder 2 steht, oder

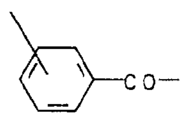

bedeutet;

$R^1$ $-(CH_2)_n-NH-x$, wobei n für eine ganze Zahl von 1 bis 6 steht, $-(CH_2)_p-C_6H_4-NH-X$, $-(CH_2)_p-C_6H_4-C(=NH)-NH_2$ oder $-(CH_2)_p-C_6H_4-CH_2-NH-X$, wobei p jeweils für 1 oder 2 steht, bedeutet, wobei aber auch anstelle von

$>CH-R^1$ $\quad$ $>C=CH-C_6H_4-X^1$

stehen kann;

$X^1$ $-NHX$, $-CH_2NHX$ oder $-C(=NH)-NH_2$ bedeutet;

X Wasserstoff, $(C_1-C_6)$-Alkyl oder einen Rest der Formel II

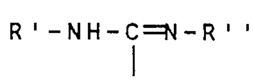

(II)

wobei R' und R" unabhängig voneinander für Wasserstoff oder $(C_1-C_6)$-Alkyl stehen, bedeutet;

$R^2$ Wasserstoff oder $(C_1-C_6)$-Alkyl bedeutet;

$R^3$ Wasserstoff oder Phenyl bedeutet;

$R^4$ Wasserstoff oder $-CO-NH-R^5$ bedeutet,

wobei NH-$R^5$ für einen α-Aminosäurerest oder dessen ω-Amino-($C_2$-$C_8$)-alkylamid steht; sowie deren physiologisch verträgliche Salze.

Alkylreste können geradkettig oder verzweigt sein. Bevorzugte Alkylreste sind Methyl, Ethyl, n-Propyl, i-Propyl, n-Butyl, i-Butyl, sek-Butyl und tert.-Butyl. Entsprechendes gilt für Reste wie Alkoxy, Alkoxycarbonyl oder Aralkyl.

Natürliche und unnatürliche α-Aminosäuren können, falls chiral, in der D- oder L-Form vorliegen. Beispielsweise seien genannt (vgl. Houben-Weyl, Methoden der organischen Chemie, Band XV/1 und 2, Stuttgart, 1974):

Aad, Abu, Aca, Ach, Acp, Aib, Ala, ΔAla, Alg, All, Ama, Apm, Apr, Arg, Asn, Asp, Asu, Aze, Azi, Bph, Can, Cit, Cys, $(Cys)_2$, Cyta, Daad, Dab, Dadd, Dap, Dapm, Dasu, Djen, Dpa, Dtc, Fel, Gln, Glu, Gly, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Iva, Lant, Lcn, Leu, Lys, ΔLys, Met, Min, nArg, Nle, Nva, Oly, Orn, Pan, Pec, Pen, Phe, Phg, Pic, Pro, ΔPro, Pse, Pya, Pyr, Pza, Sar, Sec, Sem, Ser, Thi, Thr, Thy, Thx, Tia, Tle, Tly, Trp, Trta, Tyr, Val, Tbg, Npg, Chg, Cha, Thia, 2,2-Diphenylaminoessigsäure, 2-(p-Tolyl)-2-phenylaminoessigsäure, 2-(p-Chlorphenyl)aminoessigsäure.

Ferner können die natürlichen oder unnatürlichen Aminosäuren auch als Ester bzw. Amide vorliegen, wie z. B. Methylester, Ethylamid, Semicarbazid oder ω-Amino-($C_4$-$C_8$)-alkylamid.

Funktionelle Gruppen der Aminosäuren können geschützt vorliegen. Geeignete Schutzgruppen wie z. B. Urethanschutzgruppen, Carboxylschutzgruppen und Seitenkettenschutzgruppen sind bei Hubbuch, Kontakte (Merck) 1979, Nr. 3, Seiten 14 bis 23 und bei Büllesbach, Kontakte (Merck) 1980, Nr. 1, Seiten 23 bis 35 beschrieben. Insbesondere seien genannt: Aloc, Pyoc, Fmoc, Tcboc, Z, Boc, Ddz, Bpoc, Adoc, Msc, Moc, $Z(NO_2)$, $Z(Hal_n)$, Bobz, Iboc, Adpoc, Mboc, Acm, tert.-Butyl, OBzl, ONbzl, OMbzl, Bzl, Mob, Pic, Trt.

Physiologisch verträgliche Salze der Verbindungen der allgemeinen Formel I sind insbesondere pharmazeutisch verwendbare oder nicht-toxische Salze.

Solche Salze werden beispielsweise von Verbindungen der allgemeinen Formel I, welche saure Gruppen, z. B. Carboxy, enthalten, mit Alkali- oder Erdalkalimetallen gebildet, wie z. B. Na, K, Mg und Ca, sowie mit physiologisch verträglichen organischen Aminen, wie z. B. Triethylamin und Tris-(2-hydroxy-ethyl)-amin.

Verbindungen der allgemeinen Formel I, welche basische Gruppen, z. B. eine Aminogruppe oder eine Guanidinogruppe enthalten, bilden mit anorganischen Säuren, wie z. B. Salzsäure, Schwefelsäure oder Phosphorsäure und mit organischen Carbon- oder Sulfonsäuren, wie z. B. Essigsäure, Citronensäure, Benzoesäure, Maleinsäure, Fumarsäure, Weinsäure und p-Toluolsulfonsäure Salze.

Bevorzugte Verbindungen der allgemeinen Formel I sind solche, worin

$R^1$ $-CH_2-C_6H_4-NH-C(=NH)-NH_2$; $-CH_2-C_6H_4-C(=NH)-NH_2$ oder $-CH_2-C_6H_4-CH_2-NH_2$ bedeutet;

$R^2$ Wasserstoff oder Methyl bedeutet;

$R^3$ Wasserstoff bedeutet.

Für -NH-$R^5$ stehende α-Aminosäurereste sind besonders bevorzugt der Valin-, Phenylalanin- oder der Phenylglycin-Rest.

Ein besonders bevorzugtes ω-Amino-($C_2$-$C_8$)-alkylamid ist das 4-Aminobutylamid.

Die erfindungsgemäßen Verbindungen der allgemeinen Formel I können hergestellt werden durch Fragmentkondensation einer Verbindung der allgemeinen Formel III

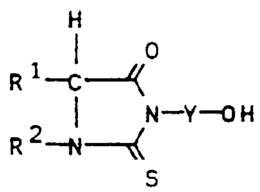

(III)

mit einer Verbindung der allgemeinen Formel IV

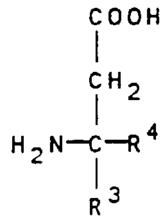

(IV)

wobei die Reste $R^1$ bis $R^4$ und Y wie oben angegeben definiert sind.

Zur Kondensation der Verbindungen der allgemeinen Formel III mit denen der allgemeinen Formel IV verwendet man vorteilhafterweise die an sich bekannten Methoden der Peptidchemie (siehe z. B. Houben Weyl, Methoden der organischen Chemie, Band 15/1 und 15/2, Stuttgart, 1974).

Dazu ist es in der Regel nötig, daß in $R^1$ und $R^4$ enthaltene Aminogruppen durch reversible Schutzgruppen geschützt werden. Gleiches gilt für die Carboxylgruppen der Verbindung der allgemeinen Formel IV, die bevorzugt als Benzyl- oder tert.-Butylester vorliegen. Ein Aminogruppen-Schutz erübrigt sich, wenn die zu generierenden Aminogruppen als Nitro- oder Cyanogruppen vorliegen und erst nach der Kupplung durch Hydrierung gebildet werden.

Nach der Kupplung werden die vorhandenen Schutzgruppen in geeigneter Weise abgespalten. Beispielsweise können $NO_2$-Gruppen (Guanidinoschutz), Benzyloxycarbonylgruppen und Benzylester abhydriert werden. Schutzgruppen vom tert.-Butyltyp werden sauer gespalten, während der 9-Fluorenylmethyloxycarbonylrest durch sekundäre Amine entfernt wird.

Die Ausgangsverbindungen der allgemeinen Formel III können wie folgt erhalten werden:

Durch Umsetzung von Aminosäuren, N-Alkylaminosäuren oder bevorzugt deren Methyl-, Ethyl-, Benzyl- oder tert.-Butylester, beispielsweise eine Verbindung der allgemeinen Formel V

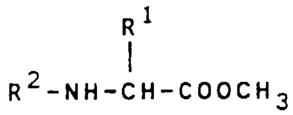

(V)

mit einem Isothiocyanatoalkancarbonsäureester, beispielsweise der allgemeinen Formel VI

$$S{=}C{=}N\text{-}(CH_2)_m\text{-}COOCH_3 \qquad (VI)$$

worin $R^1$, $R^2$ und m wie oben angegeben definiert sind, erhält man Thioharnstoffderivate, beispielsweise der allgemeinen Formel VII

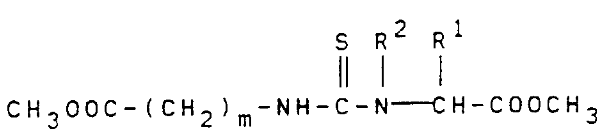

(VII),

die durch Erhitzen mit Säure unter Verseifung der Esterfunktionen zu Verbindungen der allgemeinen Formel IIIa

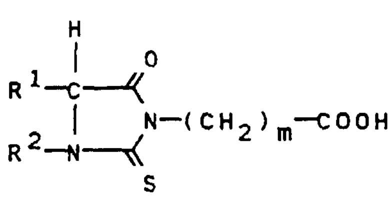

(IIIa)

cyclisieren.

Während der Thioharnstoffsynthese können Guanidinogruppen durch Schutzgruppen, wie $NO_2$ oder Mtr, blockiert

werden. Ebenso müssen Aminogruppen in der Seitenkette in geschützter Form (beispielsweise als Boc- oder Z-Derivate) oder noch als $NO_2$- oder Cyanofunktion vorliegen, die später zur Aminogruppe reduziert oder im Falle der Cyanogruppe auch in die Formamidinogruppe umgewandelt werden kann.

Verbindungen der allgemeinen Formel IIIb

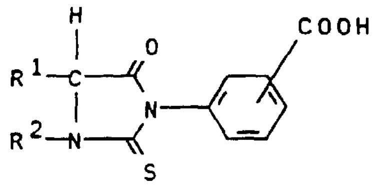

(IIIb)

können analog erhalten werden, wenn anstelle von Isothiocyanatoalkancarbonsäureestern die Isothiocyanate der Aminobenzoesäureester eingesetzt werden.

Verbindungen der allgemeinen Formel IIIc

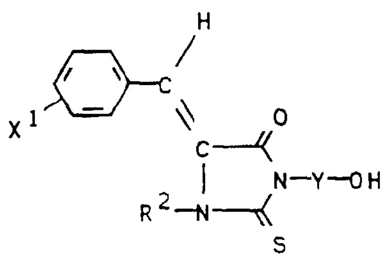

(IIIc)

können durch Umsetzung von Thiohydantoinen der allgemeinen Formel VIII

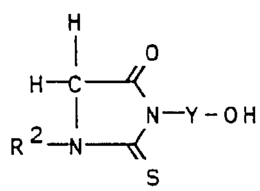

(VIII)

mit Aldehyden der allgemeinen Formel IX

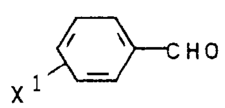

(IX)

analog Gränacher und Landolt, Helv. Chim. Acta 10 (1927) 808 erhalten werden.

Die Guanylierung der Aminofunktion kann mit folgenden Reagentien durchgeführt werden:

1. O-Methylisoharnstoff (S. Weiss und H. Krommer, Chemiker Zeitung 98 (1974) 617 - 618),

2. S-Methylisothioharnstoff (R. F. Borne, M. L. Forrester und I. W. Waters, J. Med. Chem. 20 (1977) 771 - 776),

3. Nitro-S-Methylisothioharnstoff (L. S. Hafner und R. E. Evans, J. Org. Chem. 24 (1959) 1157),

4. Formamidinosulfonsäure (K. Kim, Y.-T. Lin und H. S. Mosher, Tetrah. Lett. 29 (1988) 3183 - 3186),

5. 3.5-Dimethyl-1-pyrazolyl-formamidinium-nitrat (F. L. Scott, D. G. O'Donovan und J. Reilly, J. Amer. Chem. Soc. 75 (1953) 4053 - 4054).

Formamidine können aus den entsprechenden Cyanoverbindungen durch Anlagerung von Alkoholen (z. B. Methanol oder Ethanol) in saurem wasserfreiem Medium (z. B. Dioxan, Methanol oder Ethanol) und anschließender Behandlung mit Ammoniak in Alkoholen (z. B. Isopropanol, Methanol oder Ethanol) hergestellt werden (G. Wagner, P. Richter und Ch. Garbe, Pharmazie 29 (1974) 12 - 55). Eine weitere Methode, Formamidine herzustellen, ist die Anlagerung von $H_2S$ an die Cyanogruppe, gefolgt von einer Methylierung des entstandenen Thioamids und anschließender Umsetzung mit Ammoniak (DDR-Patent Nr. 235 866).

Die Ausgangspeptide der allgemeinen Formel IV werden in der Regel vom C-terminalen Ende her stufenweise aufgebaut. Peptidknüpfungen können mit den bekannten Kupplungsmethoden der Peptidchemie durchgeführt werden.

Die Verbindungen der allgemeinen Formel I und ihre physiologisch verträglichen Salze können als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger- und Zusatzstoffen enthalten. Die Zubereitungen enthalten normalerweise etwa 0,5 bis 90 Gew% der therapeutisch wirksamen Verbindung.

Die Heilmittel können oral, z. B. in Form von Pillen, Tabletten, Lacktabletten, Dragees, Granulaten, Hart- und Weichgelatinekapseln, Lösungen, Sirupen, Emulsion oder Suspensionen oder Aerosolmischungen verabreicht werden. Die Verabreichung kann aber auch rektal, z. B. in Form von Suppositorien oder parenteral, z. B. in Form von Injektionslösungen oder Mikrokapseln, perkutan, z. B. in Form von Salben oder Tinkturen, oder nasal, z. B. in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z. B. Lactose, Maisstärke oder Derivate davon, Talk, Stearinsäure oder deren Salze etc. verwenden. Trägerstoffe für Weichgelatinekapseln und Suppositorien sind z. B. Fette, Wachse, halbfeste und flüssige Polyole, natürliche oder gehärtete Öle etc. Als Trägerstoffe für die Herstellung von Lösungen und Sirupen eignen sich z. B. Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln oder Implantate eignen sich Mischpolymerisate aus Glykolsäure und Milchsäure.

Die pharmazeutischen Präparate können neben den Wirk- und Trägerstoffen noch Zusatzstoffe, wie z. B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer physiologisch verträglichen Salze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lantano-Glykoside; Coronardilatatoren, wie Carbochromen; Dipyridamol, Nifedipin und Perhexilin; antianginöse Verbindungen, wie Isosorbiddinitrat, Isosorbidmononitrat, Glycerolnitrat, Molsidomin und Verapamil; β-Blocker, wie Propranolol, Oxprenolol, Atenolol, Metoprolol und Penbutolol. Darüberhinaus lassen sich die Verbindungen mit anderen nootrop wirksamen Substanzen, wie z. B. Piracetam, oder ZNS-aktiven Substanzen, wie Pirlindol, Sulpirid etc., kombinieren.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Applikation beträgt die Tagesdosis im allgemeinen etwa 0,01 bis 0,3 mg/kg, vorzugsweise 0,05 bis 0,1 mg/kg Körpergewicht. Die Tagesdosis wird normalerweise, insbesondere bei der Applikation größerer Mengen, in mehrere, z. B. 2, 3 oder 4 Teilverabreichungen aufgeteilt. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,2 bis 50 mg, vorzugsweise 0,5 bis 10 mg Wirkstoff der allgemeinen Formel I oder eines ihrer physiologisch verträglichen Salze pro Dosis.

Die erfindungsgemäßen Verbindungen der Formel I haben die Fähigkeit die Zell-Zell-Adhäsion zu hemmen, die auf der Interaktion von Arg-Gly-Asp-enthaltenden Proteinen, wie Fibronectin, Fibrinogen oder des von Willebrand-Faktors mit den sogenannten Integrinen beruhen. Integrine sind Transmembran-Glykoproteine, Rezeptoren für Arg-Gly-Asp-enthaltende Zellmatrix-Glykoproteine (E. Ruoslahti und M. D. Pierschbacher, Science 238 (1987) 491 - 497;

D. R. Phillips, I. F. Charo, L. V. Parise und L. A. Fitzgerald, Blood 71 (1988) 831 - 843). Außerdem hemmen sie die Bindung weiterer adhäsiver Proteine, wie Vitronectin, Kollagen und Laminin an die entsprechenden Rezeptoren auf der Oberfläche verschiedener Zelltypen.

Die erfindungsgemäßen Verbindungen der allgemeinen Formel I hemmen die Thrombozytenaggregation, die Metastasierung von Karzinomzellen sowie die Osteoclastenbindung an die Knochenoberflächen.

Die Thiohydantoinderivate der allgemeinen Formel I finden akut Anwendung bei Thrombosegefahr und chronisch bei der Prävention der Arteriosklerose und Thrombose, z. B. bei der Therapie und Prophylaxe arterieller Gefäßerkrankungen, wie bei akutem Myokardinfarkt, Sekundärprävention des Myokardinfarkts, Reokklusionsprophylaxe nach Lyse und Dilatation (PTCA), instabiler Angina pectoris, transitorischen ischämischen Attacken, Schlaganfall, koronarer Bypass-Operation einschließlich Reokklusionsprophylaxe bei Bypass, Lungenembolie, peripherer arterieller Verschlußkrankheit, Dissezierendem Aneurysma; bei der Therapie venöser und mikrozirkulatorischer Gefäßerkrankungen, wie tiefer Venenthrombose, disseminenter intravaskulärer Gerinnung, postoperativem und post-partum Trauma, chirurgischem oder infektiösem Schock, Septicämie oder bei Erkrankungen mit hyperreagiblen Thrombozyten, thrombotischer thrombozytopenischer Purpura, Preeklampsie, prämenstruellem Syndrom, Dialyse oder extrakorporaler Zirkulation; eine weitere Anwendung ist während Krebsoperationen und auch prophylaktisch bei Krebs gegeben. Ferner kann Osteoporose durch Hemmung der Osteoclastenbindung an die Knochenoberfläche verhindert werden.

Geprüft werden die Verbindungen vor allem auf ihre hemmende Wirkung bei der Blutplättchenaggregation und der Anhaftung von Fibrinogen an Blutplättchen. Verwendet werden gefiltrierte Blutplättchen aus humanem Spenderblut, die mit ADP oder Thrombin aktiviert werden.

**Beispiele:**

Die Produkte wurden über Massenspektren und/oder NMR-Spektren identifiziert.

**Beispiel 1:**

**(5-(S)-(3-Guanidinopropyl)-4-oxo-2-thioxoimidazolidin-3-yl)-acetyl-L-aspartyl-L-phenylglycin**

**1a: Z-Arg-(Mtr)-$OCH_3$**

Zu einer Suspension von 8,5 g (16 mmol) Z-Arg-(Mtr)-OH in 100 ml Methanol werden bei 0 °C langsam 1,5 ml (20 mmol) Thionylchlorid zugetropft. Man läßt auf Raumtemperatur erwärmen und rührt 15 Stunden weiter. Nach dem Einengen (10,2 g) wird gefriergetrocknet und direkt weiter umgesetzt.

**1b: H-Arg-(Mtr)-$OCH_3$-hydrochlorid**

10 g (18,7 mmol) Z-Arg-(Mtr)-$OCH_3$ werden in 150 ml Methanol gelöst. Nach Zugabe von 1 g 10 %-Pd auf Kohle wird bei Raumtemperatur gerührt und durch Zutropfen von methanolischer Salzsäure der pH-Wert auf 4,5 eingestellt. Der Katalysator wird abfiltriert und das Filtrat eingeengt.
Ausbeute: 8 g

**1c: N-(1-Methoxycarbonyl-2(S)-(3-Mtr-guanidino-propyl)-ethyl),N'-methoxycarbonylmethyl-thioharnstoff**

5 g (11,4 mmol) H-Arg-(Mtr)-$OCH_3$-hydrochlorid werden in 40 ml Dimethylformamid gelöst. Nach Zugabe von 1,45 ml (11,4 mmol) N-Ethylmorpholin werden langsam 1,5 g (11,4 mmol) Isothiocyanatoessigsäuremethylester zugetropft. Man rührt 15 Stunden bei Raumtemperatur, engt ein, löst in Methylenchlorid und extrahiert mit einer verdünnten Kaliumhydrogensulfatlösung. Nach dem Trocknen wird die organische Lösung eingeengt.
Ausbeute: 4,4 g (72 %)

**1d: (5-(S)-(3-Guanidinopropyl)-4-oxo-2-thioxoimidazolidin-3-yl)-essigsäure**

4 g (7,5 mol) N-(1-Methoxycarbonyl-2(S)-(3-Mtr-guanidino-propyl)-ethyl),N'-methoxycarbonylmethyl-thioharnstoff werden in 40 ml 6 N Salzsäure 20 Minuten unter Rückfluß erhitzt. Dann wird eingeengt, mit Wasser/ Methanol versetzt, vom unlöslichen Rückstand abgetrennt und eingeengt. Die Substanz wird zur Reinigung an Sephadex LH20 mit einer homogenen Mischung von Butanol/Eisessig/ Wasser chromatographiert.
Ausbeute 1,9 g (92 %)

**1e: (5-(S)-(3-Guanidinopropyl)-4-oxo-2-thioxoimidazolidin-3-yl)-acetyl-L-aspartyl(OtBu)-L-phenylglycin-OtBu**

Zu einer Lösung von 300 mg (1,1 mmol) (5-(S)-(3-Guanidinopropyl)-4-oxo-2-thioxoimidazolidin-3-yl)-essigsäure, 472 mg (1,1 mmol) H-Asp(OtBu)-phenylglycin-OtBu-hydrochlorid, 148 mg (1,1 mmol) Hydroxybenzotriazol in 10 ml Dimethylformamid gibt man bei 0 °C 127 mg (1,1 mmol) N-Ethylmorpholin und 250 mg (1,21 mmol) DCC.

Man rührt 1 Stunde bei 0 °C und anschließend 5 Stunden bei Raumtemperatur. Der ausgefallene Harnstoff wird abgesaugt, das Filtrat eingeengt und das Rohprodukt direkt weiter umgesetzt.

**1f: (5-(S)-(3-Guanidinopropyl)-4-oxo-2-thioxoimidazolidin-3-yl)-acetyl-L-aspartyl-L-phenylglycin**

Man läßt 700 mg (5-(S)-(3-Guanidinopropyl)-4-oxo-2-thioxoimidazolidin-3-yl)-acetyl-L-aspartyl(OtBu)-L-phenylglycin-OtBu mit 5 ml 95 proz. Trifluoressigsäure unter gelegentlichem Umschütteln 3 Stunden bei Raumtemperatur stehen und engt ein. Das Rohprodukt wird zur Reinigung an Sephadex LH20 mit einer homogenen Mischung von Butanol/Eisessig/Wasser chromatographiert.
Ausbeute 222 mg
Schmelzpunkt 170 °C
$(\alpha)_D^{25}$ = 11,8° (c = 0,255, Wasser)
Massenspektrum: M + 1-peak bei 522

**Beispiel 2:**

**(5-(S,R)-(4-Formamidino-benzyl)-4-oxo-2-thioxoimidazolidin-3-yl)-acetyl-L-aspartyl-L-phenylglycin**

**2a:**
**N-(2-(4-Formamidino-phenyl)-1-methoxycarbonyl-ethyl),N'-methoxycarbonylmethyl-thioharnstoff**

Zu einer Lösung von 2.93 g (10 mmol) 4-Formamidino-phenylalanin-methylester-dihydrochlorid und 1,3 g (10 mmol) Isothiocyanatoessigsäuremethylester in 25 ml Dimethylformamid läßt man bei Raumtemperatur langsam 1,3 ml (10 mmol) N-Ethylmorpholin in 3 ml Dimethylformamid zutropfen. Um die Umsetzung zu vervollständigen werden später nochmals 0.35 ml Isothiocyanatoessigsäuremethylester zugegeben. Man läßt über das Wochenende bei +4°C stehen, engt ein und chromatographiert zur Reinigung an Sephadex LH20 mit einer homogenen Mischung von Butanol/Eisessig/Wasser.
Ausbeute: 2,7 g

**2b:**
**(5-(S,R)-(4-Formamidino-benzyl)-4-oxo-2-thioxoimidazolidin-3-yl)-essigsäure**

2,5 g N-(2-(4-Formamidino-phenyl)-1-methoxycarbonylethyl),N'-methoxycarbonylmethyl-thioharnstoff werden in 10 ml 6 N Salzsäure 30 Minuten unter Rückfluß erhitzt. Dann wird eingeengt und der Rückstand mit 50 ml Wasser versetzt. Man stellt mit $NaHCO_3$ einen pH von 5-6 ein und läßt über Nacht bei 4°C stehen. Anderntags wird der Niederschlag abgesaugt, mit wenig kaltem Wasser gewaschen und in Hochvakuum getrocknet.
Ausbeute: 1,79 g
Schmelzpunkt: 280-283°C (Zers.)

**2c: (5-(S,R)-(4-Formamidino-benzyl)-4-oxo-2-thioxoimidazolidin-3-yl)-acetyl-L-aspartyl-L-phenylglycin-di-tert.-butylester-hydrochlorid**

Zu einer Suspension von 650 mg (2 mmol) (5-(S,R)-(4-Formamidino-benzyl)-4-oxo-2-thioxoimidazo lidin-3-yl)-essigsäure, 830 mg H-Asp(OtBu)-phenylglycin-OtBu-hydrochlorid, 270 mg Hydroxybenzotriazol in 4 ml Dimethylformamid gibt man bei 0°C 430 mg DCC. Man rührt 1 Stunde bei 0°C und anschließend 2 Stunden bei Raumtemperatur. Der ausgefallene Harnstoff wird abgesaugt, das Filtrat eingeengt und das Rohprodukt über Kieselgel in einer Mischung aus Methylenchlorid, Methanol, Eisessig und Wasser wie 90:10:1:1 chromatographiert.
Ausbeute: 1,25 g

**2d: (5-(S,R)-(4-Formamidino-benzyl)-4-oxo-2-thioxoimidazolidin-3-yl)-acetyl-L-aspartyl-L-phenylglycin**

1.2 g (5-(S,R)-(4-Formamidino-benzyl)-4-oxo-2-thioxoimidazolidin-3-yl)-acetyl-L-aspartyl-L-phenylglycin-ditert. -butylester-hydrochlorid werden mit 15 ml 90 proz. Trifluoressigsäure und 1.5 ml 1,2 Dimercaptoethanol 1 Stunde bei Raumtemperatur gerührt und eingeengt. Der Rückstand wird zwischen Ether und Wasser verteilt. Die wässrige Phase wird gefriergetrocknet und zur Reinigung an Sephadex LH20 mit einer homogenen Mischung von Butanol/Eisessig/Wasser zweimal chromatographiert.
Ausbeute: 280 mg
$(\alpha)_D^{26}$ = +15.7° (c = 1, 90-proz. Essigsäure)

Analog der oben beschriebenen Beispiele können die folgenden Verbindungen hergestellt werden:

**Beispiel 3:**

**3-(5-(S)-(3-Aminopropyl)-4-oxo-2-thioxoimidazolidin-3-yl) -benzoyl-L-aspartyl-L-phenylglycin**

FAB-MS 542 $(M+H)^+$

**Beispiel 4:**

**(5-(4-Guanidino-benzyl)-oxo-2-thioxoimidazolidin-3-yl)-acetyl-L-aspartyl-L-valin**

FAB-MS 536 $(M+H)^+$

**Beispiel 5:**

**(5-(4-Formamidinobenzyliden)-4-oxo-2-thioxoimidazolidin-3-yl)-acetyl-L-aspartyl-L-tryptophan**

FAB-MS 606 $(M+H)^+$

**Beispiel 6:**

**3-(5-(S,R)-(4-Formamidino-benzyl)-4-oxo-2-thioxoimidazolidin-3-yl)-propionyl-L-aspartyl-L-phenylalanin-(4-amino-butyl)-amid**

FAB-MS 653 $(M+H)^+$

**Beispiel 7:**

**(5-(4-Aminomethylbenzyliden)-4-oxo-2-thioxoimidazolidin-3-yl)-acetyl-L-aspartyl-L-lysin**

FAB-MS 535 $(M+H)^+$

**Beispiel 8:**

**3-(5-Guanidinomethyl-4-oxo-2-thioxoimidazolidin-3-yl)-benzoyl-L-aspartyl-D-phenylglycin**

FAB-MS 556 $(M+H)^+$

Beispiel A

Emulsionen mit 3 mg Wirkstoff per 5 ml können nach folgender Rezeptur hergestellt werden:

| | |
|---|---|
| Wirkstoff | 0,06 g |
| Neutralöl | q. s. |
| Natriumcarboxymethylzellulose | 0,6 g |
| Polyoxyethylenstearat | q. s. |
| Reinglycerin | 0,6 bis 2 g |
| Aromastoffe | q. s. |
| Wasser (entmineralisiert oder destilliert) | ad 100 ml |

Beispiel B

Tabletten können nach folgender Formulierung hergestellt werden:

| | |
|---|---|
| Wirkstoff | 2 mg |
| Lactose | 60 mg |
| Maisstärke | 30 mg |

(fortgesetzt)

| | |
|---|---|
| lösliche Stärke | 4 mg |
| Magnesiumstearat | 4 mg |
| | 100 mg |

Beispiel C

Für die Herstellung von Weichgelatinekapseln mit 5 mg Wirkstoff pro Kapsel eignet sich die folgende Zusammensetzung:

| | |
|---|---|
| Wirkstoff | 5 mg |
| Mischung von Triglyceriden aus Kokosöl | 150 mg |
| Kapselinhalt | 155 mg |

Beispiel D

Für die Herstellung von Dragees eignet sich folgende Formulierung:

| | |
|---|---|
| Wirkstoff | 3 mg |
| Maisstärke | 100 mg |
| Lactose | 55 mg |
| sec. Calciumphosphat | 30 mg |
| lösliche Stärke | 3 mg |
| Magnesiumstearat | 5 mg |
| kolloidale Kieselsäure | 4 mg |
| | 200 mg |

Beispiel E

Dragees, enthaltend einen erfindungsgemäßen Wirkstoff und einen anderen therapeutisch wirksamen Stoff:

| | |
|---|---|
| Wirkstoff | 6 mg |
| Propanolol | 40 mg |
| Milchzucker | 90 mg |
| Maisstärke | 90 mg |
| sec. Calciumphosphat | 34 mg |
| lösliche Stärke | 3 mg |
| Magnesiumstearat | 3 mg |
| kolloidale Kieselsäure | 4 mg |
| | 270 mg |

Beispiel F

Dragees, enthaltend einen erfindungsgemäßen Wirkstoff und einen anderen therapeutisch wirksamen Stoff:

| | |
|---|---|
| Wirkstoff | 5 mg |
| Pirlindol | 5 mg |
| Milchzucker | 60 mg |
| Maisstärke | 90 mg |
| sec. Calciumphosphat | 30 mg |
| lösliche Stärke | 3 mg |

(fortgesetzt)

| | |
|---|---|
| Magnesiumstearat | 3 mg |
| kolloidale Kieselsäure | 4 mg |
| | 200 mg |

Beispiel G

Kapseln, enthaltend einen erfindungsgemäßen Wirkstoff und einen anderen therapeutisch wirksamen Stoff:

| | |
|---|---|
| Wirkstoff | 5 mg |
| Nicergolin | 5 mg |
| Maisstärke | 185 mg |
| | 195 mg |

Beispiel H

Injektionslösungen mit 1 mg Wirkstoff pro ml können nach folgender Rezeptur hergestellt werden:

| | |
|---|---|
| Wirkstoff | 1,0 mg |
| Polyethylenglykol 400 | 0,3 mg |
| Natriumchlorid | 2,7 mg |
| Wasser zu Injektionszwecken auf | 1 ml |

Pharmakologische Daten:

Geprüft wird die Hemmung der Bindung von Fibrinogen an seinen Rezeptor (Glykoprotein IIb/IIIa) an intakten, gelfiltrierten Human-Thrombozyten durch die erfindungsgemäßen Verbindungen. Angegeben ist der $K_i$ Wert der Bindungshemmung von $^{125}$I-Fibrinogen nach Stimulierung mit ADP (10µM).

Literatur:

J.S. Bennett u. G. Vilaire, J. Clin. Invest. 64 (1979), 1393-1401
E. Kornecki et al., J. Biol. Chem. 256 (1981), 5695-5701 G.A. Marguerie et al., J. Biol. Chem. 254 (1979), 5357-5363
G.A. Marguerie et al., J. Biol. Chem. 255 (1980), 154-161

| Beispiel | $K_i$ (µM), ADP stimuliert |
|---|---|
| 1 | 0,07 |
| 2 | 0,2 |

Als funktioneller Test wird die Hemmung der Aggregation gelfiltrierter Human-Thrombozyten nach ADP- oder Thrombin-Stimulierung durch die erfindungsgemäßen Verbindungen gemessen. Angegeben ist der $IC_{50}$-Wert der Hemmung.

Literatur:

G.A. Marguerie et al., J. Biol. Chem. 254 (1979), 5357-5363

| Beispiel | ADP-stimuliert | $IC_{50}$ (µM), Thrombin-stimuliert |
|---|---|---|
| 1 | 0,5 | 0,1 |
| 2 | 0,55 | 0,2 |

## Patentansprüche

1. Verbindungen der allgemeinen Formel I

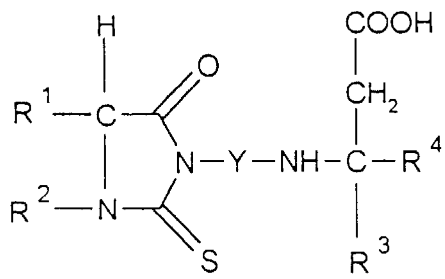

(I)

worin

Y $-(CH_2)_m-CO-$, wobei m für 1 oder 2 steht, oder

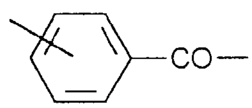

bedeutet;

$R^1$ $-(CH_2)_n-NH-X$, wobei n für eine ganze Zahl von 1 bis 6 steht, $-(CH_2)_p-C_6H_4-NH-X$, $-(CH_2)_p-C_6H_4-C(=NH)-NH_2$ oder $-(CH_2)_p-C_6H_4-CH_2-NH-X$, wobei p jeweils für 1 oder 2 steht, bedeutet, wobei aber auch anstelle von

$>CH—R^1$ $\quad$ $>C=CH-C_6H_4-X^1$

stehen kann;

$X^1$ $-NHX$, $-CH_2NHX$ oder $-C(=NH)-NH_2$ bedeutet;

X Wasserstoff, $(C_1-C_6)$-Alkyl oder einen Rest der Formel II

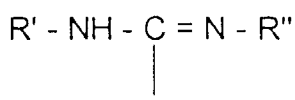

(II)

wobei R' und R" unabhängig voneinander für Wasserstoff oder $(C_1-C_6)$-Alkyl stehen, bedeutet;

$R^2$ Wasserstoff oder $(C_1-C_6)$-Alkyl bedeutet;

$R^3$ Wasserstoff oder Phenyl bedeutet;

$R^4$ Wasserstoff oder $-CO-NH-R^5$ bedeutet, wobei $-NH-R^5$ für einen α-Aminosäurerest oder dessen ω-Amino-$(C_2-C_8)$-alkylamid steht;

sowie deren physiologisch verträgliche Salze.

2. Verbindungen gemäß Anspruch 1, dadurch gekennzeichnet, daß in der allgemeinen Formel I

$R^1$ $-CH_2-C_6H_4-NH-C(=NH)-NH_2$; $-CH_2-C_6H_4-C(=NH)-NH_2$ oder $-CH_2-C_6H_4-CH_2-NH_2$ bedeutet;

$R^2$ Wasserstoff oder Methyl bedeutet; und

$R^3$ Wasserstoff bedeutet.

**3.** Verbindungen gemäß Anspruch 1 und/oder 2, dadurch gekennzeichnet, daß für -NH-$R^5$ stehende Aminosäurereste der Valin-, Phenylalanin- oder der Phenylglycin-Rest sind.

**4.** Verbindungen gemäß einem oder mehreren der Ansprüche 1 bis 3, dadurch gekennzeichnet, daß das ω-Amino-($C_2$-$C_8$)-alkylamid das 4-Aminobutylamid ist.

**5.** Verfahren zur Herstellung von Verbindungen der Ansprüche 1 bis 4, dadurch gekennzeichnet, daß man eine Fragmentkondensation einer Verbindung der allgemeinen Formel III

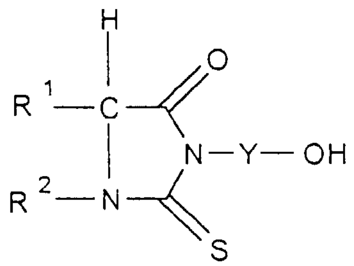

( III )

mit einer Verbindung der allgemeinen Formel IV

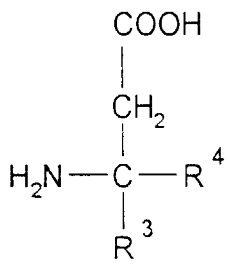

( IV )

wobei die Reste $R^1$ bis $R^4$ und Y wie in Anspruch 1 angegeben definiert sind, ausführt.

**6.** Verbindung der allgemeinen Formel I gemäß einem oder mehreren der Ansprüche 1 bis 4 zur Anwendung als Hemmstoff der Thrombozytenaggregation, der Metastasierung von Karzinomzellen sowie der Osteoclastenbindung an die Knochenoberflächen.

**7.** Pharmazeutisches Präparat, dadurch gekennzeichnet, daß es eine oder mehrere Verbindungen der allgemeinen Formel I der Ansprüche 1 bis 4 oder ein physiologisch verträgliches Salz davon als Wirkstoff zusammen mit pharmazeutisch annehmbaren Träger- und Zusatzstoffen und gegebenenfalls noch ein oder mehrere andere pharmakologische Wirkstoffe enthält.

**8.** Verfahren zur Herstellung eines pharmazeutischen Präparates, enthaltend eine oder mehrere Verbindungen der allgemeinen Formel I der Ansprüche 1 bis 4 oder ein physiologisch verträgliches Salz davon, dadurch gekennzeichnet, daß man diese zusammen mit pharmazeutisch annehmbaren Träger- und Zusatzstoffen und gegebenenfalls noch ein oder mehreren anderen pharmakologischen Wirkstoffen in eine geeignete Darreichungsform bringt.

## Claims

1. Compounds of the general formula I

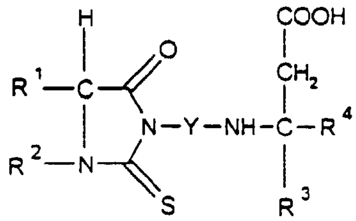

(I)

in which

Y denotes $-(CH_2)_m-CO-$, where m stands for 1 or 2, or

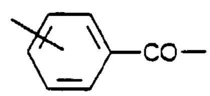

;

$R^1$ denotes $-(CH_2)_n-NH-X$, where n stands for a whole number from 1 to 6, $-(CH_2)_p-C_6H_4-NH-X$, $-(CH_2)_p-C_6H_4-C(=NH)-NH_2$ or $-(CH_2)_p-C_6H_4-CH_2-NH-X$, where p in each case stands for 1 or 2,
where, however >CH-$R^1$ can also be replaced by >$C=CH-C_6H_4-X^1$;
$X^1$ denotes $-NHX$, $-CH_2NHX$ or $-C(=NH)-NH_2$;
X denotes hydrogen, $(C_1-C_6)$-alkyl or a radical of the formula II

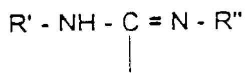

(II)

where R' and R", independently of each other, stand for hydrogen or $(C_1-C_6)$-alkyl;
$R^2$ denotes hydrogen or $(C_1-C_6)$-alkyl;
$R^3$ denotes hydrogen or phenyl;
$R^4$ denotes hydrogen or $-CO-NH-R^5$, where $-NH-R^5$ stands for an $\alpha$-amino acid residue or its $\omega$-amino-$(C_2-C_8)$-alkylamide;

as well as their physiologically tolerated salts.

2. Compounds according to Claim 1, characterised in that in the general formula I

$R^1$ denotes $-CH_2-C_6H_4-NH-C(=NH)-NH_2$; $-CH_2-C_6H_4-C(=NH)-NH_2$ or $-CH_2-C_6H_4-CH_2-NH_2$;
$R^2$ denotes hydrogen or methyl; and
$R^3$ denotes hydrogen.

3. Compounds according to Claim 1 and/or 2, characterised in that amino acid residues standing for $-NH-R^5$ are the valine residue, the phenylalanine residue or the phenylglycine residue.

4. Compounds according to one or more of Claims 1 to 3, characterised in that the $\omega$-amino-$(C_2-C_8)$-alkylamide is the 4-aminobutylamide.

5. Process for preparing compounds of Claims 1 to 4, characterised in that a fragment condensation of a compound of the general formula III

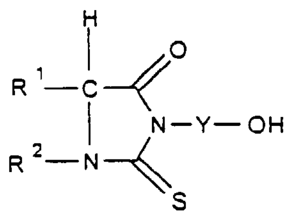

(III)

with a compound of the general formula IV

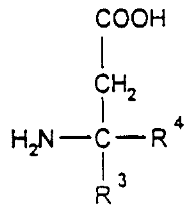

(IV)

where the radicals $R^1$ to $R^4$ and Y are defined as indicated in Claim 1, is carried out.

6. Compound of the general formula I according to one or more of Claims 1 to 4 for use as an inhibitor of platelet aggregation, of the metastasis of carcinoma cells and of the binding of osteoclasts to the bone surfaces.

7. Pharmaceutical product, characterized in that it comprises one or more compounds of the general formula I of Claims 1 to 4 or a physiologically tolerated salt thereof as active substance together with pharmaceutically acceptable excipients and additives and optionally one or more other pharmacological active substances in addition.

8. Process for the preparation of a pharmaceutical product comprising one or more compounds of the general formula I of Claims 1 to 4 or a physiologically tolerated salt thereof, characterized in that the latter is/are brought into a suitable administration form together with pharmaceutically acceptable excipients and additives and optionally one or more other pharmacological active substances in addition.

## Revendications

1. Composés de formule générale I

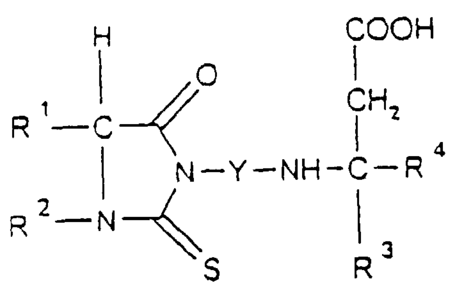

(I)

dans laquelle

Y représente $-(CH_2)_m-CO-$, m représentant 1 ou 2, ou

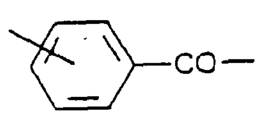

$R^1$ représente un groupe $-(CH_2)_n-NH-X$, n représentant un nombre entier allant de 1 à 6,$-(CH_2)_p-C_6H_4-NH-X$, $-(CH_2)_p-C_6H_4-C(=NH)-NH_2$ ou $(CH_2)_p-C_6H_4-CH_2-NH-X$, p représentant dans chaque cas 1 ou 2, un groupe $>C=CH-C_6H_4-X^1$ pouvant toutefois être présent au lieu de $>CH-R^1$;
$X^1$ représente $-NHX$, $-CH_2NHX$ ou $-C(=NH)-NH_2$;
X représente un atome d'hydrogène, un groupe alkyle en $C_1$-$C_6$ ou un radical de formule II

$$R'-NH-C=N-R'' \qquad (II)$$

R' et R" représentant, indépendamment l'un de l'autre, un atome d'hydrogène ou un groupe alkyle en $C_1$-$C_6$;
$R^2$ représente un atome d'hydrogène ou un groupe alkyle en $C_1$-$C_6$;
$R^3$ représente un atome d'hydrogène ou le groupe phényle;
$R^4$ représente un atome d'hydrogène ou $-CO-NH-R^5$, $NH-R^5$ représentant un reste d'a-aminoacide ou d'un ω-aminoalkyl($C_2$-$C_8$)amide de celui-ci;

ainsi que leurs sels physiologiquement acceptables.

**2.** Composés selon la revendication 1, caractérisés en ce que, dans la formule générale I,

$R^1$ représente $-CH_2-C_6H_4-NH-C(=NH)NH_2$, $-CH_2-C_6H_4-C(=NH)-NH_2$ ou $-CH_2-C_6H_4-CH_2-NH_2$;
$R^2$ représente un atome d'hydrogène ou le groupe méthyle;
$R^3$ représente un atome d'hydrogène.

**3.** Composés selon la revendication 1 ou 2, caractérisés en ce que les restes d'a-aminoacides représentés par $-NH-R^5$ sont le reste de la valine, de la phénylalanine ou de la phénylglycine.

**4.** Composés selon une ou plusieurs des revendications 1 à 3, caractérisés en ce que l'ω-amino-alkyl-($C_2$-$C_8$)amide est le 4-aminobutylamide.

**5.** Procédé pour la préparation des composés des revendications 1 à 4, caractérisé en ce que l'on effectue une condensation de fragments, en condensant un composé de formule générale III

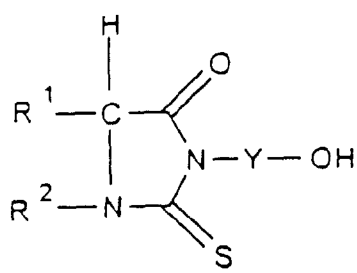

(III)

avec un composé de formule générale IV

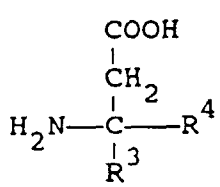

(IV)

les radicaux $R^1$ à $R^4$ et Y étant définis comme indiqué dans la revendication 1.

**6.** Composé de formule générale I selon une ou plusieurs des revendication 1 à 4, pour utilisation en tant qu'inhibiteur de l'agrégation des thrombocytes, de la dissémination de métastases de cellules cancéreuses, ainsi que de la fixation d'ostéoclastes à la surface des os.

7. Composition pharmaceutique, caractérisée en ce qu'elle contient, en tant que substance active, un ou plusieurs des composés de formule générale I selon les revendications 1 à 4, ou un(des) sel(s) physiologiquement acceptables de celui-ci(ceux-ci), conjointement avec des additifs et véhicules pharmaceutiquement acceptables et éventuellement encore une ou plusieurs autres substances pharmacologiquement actives.

8. Procédé pour la préparation d'une composition pharmaceutique, contenant un ou plusieurs des composés de formule générale I selon les revendications 1 à 4, ou un(des) sel(s) physiologiquement acceptable(s) de celui-ci (ceux-ci), caractérisé en ce qu'on le(s) met sous une forme d'administration appropriée, conjointement avec des additifs et véhicules pharmaceutiquement acceptables et éventuellement encore une ou plusieurs autres substances pharmacologiquement actives.